# EUROPEAN PATENT SPECIFICATION

(11) **EP 4 313 216 B1**
(45) Date of publication and mention of the grant of the patent: **28.10.2026**
(21) Application number: 21843697.0
(22) Date of filing: 21.12.2021
(51) Int. Cl.: A61M 5/32, A61M 5/28, A61M 5/20, A61M 5/50

(54) **MEDICAMENT DELIVERY DEVICE**
MEDIKAMENTENVERABREICHUNGSVORRICHTUNG
DISPOSITIFS D'ADMINISTRATION DE MÉDICAMENTS

(30) Priority: 31.03.2021 EP 21166428
(43) Date of publication of application: 07.02.2024
(73) Proprietor: SHL Medical AG, 6302 Zug (CH)
(72) Inventor: CARLSSON, Daniel, 131 28 Nacka Strand (SE)
(86) International application number: PCT/EP2021/086919
(87) International publication number: WO 2022/207136

(56) References cited:
- US-A- 4 019 655
- US-A1- 2003 212 362
- US-A1- 2010 137 808
- US-A1- 2014 046 270
- US-A1- 2015 209 519
- US-A1- 2017 080 153
- US-A1- 2020 345 937

## Description

### TECHNICAL FIELD

The present disclosure generally relates to a medicament delivery device for expelling medicament from a flexible medicament container.

### BACKGROUND

Some liquid chambers may be mechanically flexible or soft. A blow-fill-seal (BFS) container is an example of a common type of flexible container containing liquid.

Today, BFS containers filled with a medicament are typically handled manually by the user during administration. This means that the user has to compress the liquid chamber or the gas-filled chamber manually to expel the medicament from the BFS container. This is typically done by medical professionals to ensure that the dose is administered correctly.

US2003/0212362 A1 discloses an injector device configured to discharge the content of a plastic blister Uniject^{™} device. The injector device has a cap and a barrel configured to slide relative to the cap. A shaped plastic moulding is used to enclose the Uniject^{™} device in the injection device. During assembly, the shaped plastic moulding is folded around the Uniject^{™} device. In use, a distal free end of the device is pressed against the skin of the patient. The cap is then able to slide telescopically over the barrel causing inner tabs of the injection device to break. The shaped plastic moulding eventually slides over the Uniject^{™} device, until its dome contacts, and presses on, the blister. The content of the blister is thus discharged into the subcutaneous tissue or muscle layer.

The injection relies on the interaction as the shaped plastic moulding slides in the barrel and presses against the blister of the Uniject^{™} device. The controllability of the injection is therefore relatively low, and the blister may thus not be fully emptied.

US2015/0209519A1 discloses a drug delivery device with a spring-driven plunger that acts on a compressible medicament container through hinged plates.

### SUMMARY

An object of the present disclosure is thus to provide a medicament delivery device which solves, or at least mitigates, problems of the prior art.

The invention is defined by appended claim 1 with the dependent claims defining advantageous embodiments.

There is hence according to a first aspect of the present disclosure provided a medicament delivery device for expelling medicament from a mechanically flexible medicament container structure, the medicament delivery device comprising: a housing having a proximal end and a distal end, a hinge structure having two hinge arms, the hinge structure being arranged in the housing and configured to receive a portion of the medicament container structure between the two hinge arms, and a hollow drive member biased towards the proximal end and arranged distally relative to the hinge arms in the housing, the drive member having an inner diameter which is smaller than a radial dimension of the hinge arms, wherein the drive member is configured to be released from a first axial position during a medicament delivery operation and move towards the proximal end of the housing, causing the drive member to move radially over the hinge arms which thereby move radially inwards towards each other to mechanically deform the portion of the medicament container structure for expulsion of medicament.

An effect obtainable thereby is that improved control of medicament discharging may be provided.

In the present disclosure, when the term "distal direction" is used, this refers to the direction pointing away from the dose delivery site during use of the medicament delivery device. When the term "distal part/end" is used, this refers to the part/end of the delivery device, or the parts/ends of the components thereof, which under use of the medicament delivery device is/are located furthest away from the dose delivery site. Correspondingly, when the term "proximal direction" is used, this refers to the direction pointing towards the dose delivery site during use of the medicament delivery device. When the term "proximal part/end" is used, this refers to the part/end of the delivery device, or the parts/ends of the members thereof, which under use of the medicament delivery device is/are located closest to the dose delivery site.

Further, the term "longitudinal", "longitudinally", "axially" or "axial" refer to a direction extending from the proximal end to the distal end, typically along the device or components thereof in the direction of the longest extension of the device and/or component.

Similarly, the terms "transverse", "transversal" and "transversally" refer to a direction generally perpendicular to the longitudinal direction.

Further, the terms "circumference", "circumferential", "circumferentially" refer to a circumference or a circumferential direction relative to an axis, typically a central axis extending in the direction of the longest extension of the device and/or component. Similarly, "radial" or "radially" refer to a direction extending radially relative to the axis, and "rotation", "rotational" and "rotationally" refer to rotation relative to the axis.

According to one embodiment the hinge arms are directed towards the proximal end of the housing. The hinge arms thus extend towards the proximal end of the housing.

According to one embodiment each hinge arm has an end portion that tapers in a direction towards the distal end of the housing. The end portion may for example have the shape of a ramp directed towards the distal end of the housing.

According to one embodiment the hinge structure is biased towards the distal end of the housing, and wherein the hinge structure has a frame provided with radially outwards extending protrusions configured to bear against a proximal end of the drive member for distancing the hinge arms from contact with the drive member when the drive member is in the first axial position.

The hinge structure may thereby be provided in a fixed axial position, in which the hinge arms do not interact with the drive member prior to medicament delivery.

One embodiment comprises a distal resilient member configured to bias the drive member towards the proximal end of the housing.

According to one embodiment the mechanically flexible medicament container structure is a blow-fill-seal container.

There is according to a second aspect of the present disclosure provided a medicament delivery device for expelling medicament from a mechanically flexible medicament container structure having a proximal liquid container and a distal gas container in fluid communication with the liquid container, the medicament delivery device comprising: a housing having a proximal end and a distal end, a liquid container holder biased towards the distal end, the liquid container holder having a chamber configured to receive the liquid container, the liquid container holder having oppositely arranged radially flexible arms forming part of a respective side surface of the chamber, each arm having a radially outwards extending structure, an expulsion member configured to pressurise the gas container during a medicament delivery operation such that gas is expelled from the gas container into the liquid container to thereby expel liquid from the liquid container, and a holding sleeve arranged around the liquid container holder and being axially fixed relative to the housing, wherein the radially outwards extending structures are configured to be in contact with a respective radial surface of the holding sleeve to prevent the liquid container holder to move axially towards the distal end, wherein the arms are urged radially inwards against the liquid container by the biasing and the contact with the radial surfaces of the holding sleeve, wherein the arms are subjected to a radially outwards directed counterpressure by the liquid container when the liquid container is filled with liquid, and wherein the arms are configured to move radially inwards when the liquid container is emptied and its internal pressure is reduced, causing the radially outwards extending structures to be released from contact with the radial surfaces of the holding sleeve and the liquid container holder to move towards the distal end inside the housing and obtain a final axial position.

An indication that the medicament delivery has been completed may thereby be provided to the user. In particular, the user may be made aware of the end of delivery because the liquid container holder moves further into the housing.

Typically, the mechanically flexible medicament container structure, provided with a delivery member such as a needle, will also follow the distal movement of the liquid container holder because the liquid container is fixedly arranged in the liquid container holder. The delivery member may for example be moved fully into the housing.

According to one embodiment the radially outwards extending structures and/or the radial surfaces define inclined surfaces relative to a radial plane of the housing to facilitate radial inwards movement of the arms.

According to one embodiment the holding sleeve comprises radial openings, wherein each radial surface is formed by a distal end surface of a respective opening.

One embodiment comprises a proximal sleeve, and a proximal resilient member biasing the proximal sleeve against the liquid container holder in a direction towards the distal end of the housing, thereby biasing the liquid container holder towards the distal end of the housing.

One embodiment comprises a hinge structure having two hinge arms, the hinge structure being arranged in the housing and configured to receive the gas container between the two hinge arms, wherein the liquid container holder forms a proximal end of the hinge structure, wherein the expulsion member is a hollow drive member biased towards the proximal end and arranged distally relative to the hinge arms in the housing, the drive member having an inner diameter which is smaller than a radial dimension of the hinge arms, wherein the drive member is configured to be released from a first axial position during a medicament delivery operation and move towards the proximal end of the housing, causing the drive member to move radially over the hinge arms which thereby move radially inwards towards each other to mechanically deform the gas container.

According to one embodiment the hinge structure has a radially flexible frame provided with radially outwards extending protrusions configured to bear against a proximal end of the drive member for distancing the hinge arms from contact with the drive member when the drive member is in the first axial position, the drive member having radial openings configured to interlock with a respective one of the protrusions when the liquid container holder has reached its final axial position.

According to one embodiment the mechanically flexible medicament container structure is a blow-fill-seal container.

Generally, all terms used in the claims are to be interpreted according to their ordinary meaning in the technical field, unless explicitly defined otherwise herein. All references to "a/an/the member, apparatus, component, means, etc." are to be interpreted openly as referring to at least one instance of the member, apparatus, component, means, etc., unless explicitly stated otherwise.

### BRIEF DESCRIPTION OF THE DRAWINGS

The specific embodiments of the inventive concept will now be described, by way of example, with reference to the accompanying drawings, in which:
Fig. 1 schematically shows a perspective view of an example of a medicament delivery device;
Fig. 2 shows an exploded view of the medicament delivery device in Fig. 1;
Figs 3A-3B show a perspective view and a top view, respectively, of a hinge structure of the medicament delivery device in Fig. 2;
Fig. 4A is a sectional view of the medicament delivery device in a default state;
Fig. 4B is a sectional view of the medicament delivery device in the default state, the section being taken at 90 degrees angle relative to the section shown in Fig. 4A;
Fig. 5A is a sectional view of the medicament delivery device during a medicament delivery operation;
Fig. 5B is a sectional view of the medicament delivery device during a medicament delivery operation, the section being taken at 90 degrees angle relative to the section shown in Fig. 5A;
Fig. 6 is a sectional view of the medicament delivery device during medicament delivery, when arms collapse over a liquid container;
Fig. 7A is a sectional view of the medicament delivery in a final state after medicament delivery has been performed; and
Fig. 7B is a sectional view of the medicament delivery device in the final state, the section being taken at 90 degrees angle relative to the section shown in Fig. 7A.

### DETAILED DESCRIPTION

The inventive concept will now be described more fully hereinafter with reference to the accompanying drawings, in which exemplifying embodiments are shown. The inventive concept may, however, be embodied in many different forms and should not be construed as limited to the embodiments set forth herein; rather, these embodiments are provided by way of example so that this disclosure will be thorough and complete, and will fully convey the scope of the inventive concept to those skilled in the art. Like numbers refer to like members throughout the description.

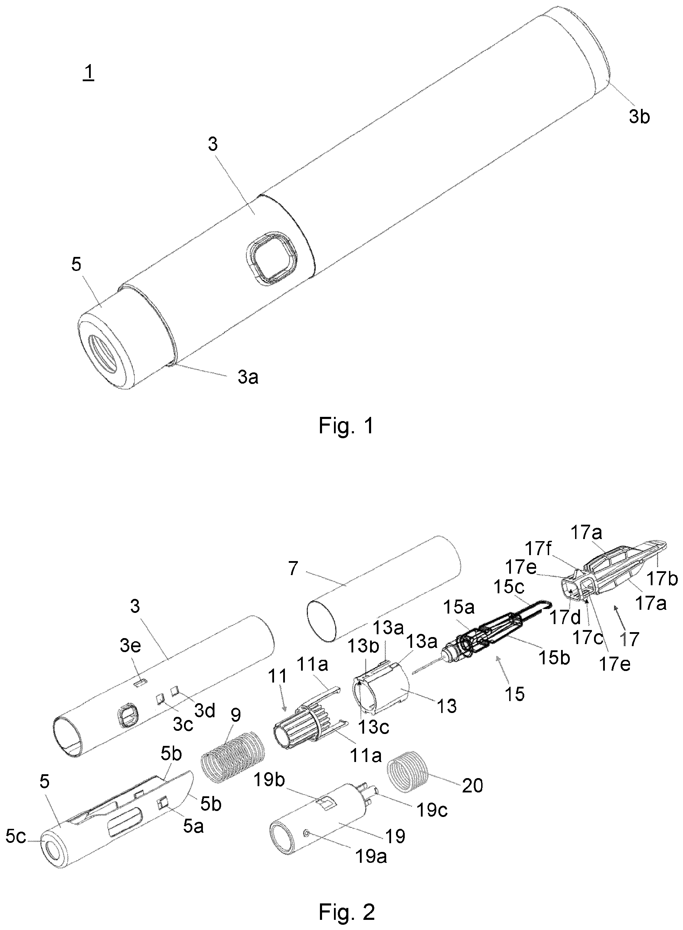

Fig. 1 shows an example of a medicament delivery device 1 configured to expel medicament from a mechanically flexible medicament container structure. The mechanically flexible medicament container structure may for example be a BFS container.

The medicament delivery device 1 comprises a housing 3. The housing 3 has a proximal end 3a and a distal end 3b.

The medicament delivery device 1 comprises a delivery member cover 5. The delivery member cover 5 is arranged in the housing 3 and extends proximally from the proximal end 3a.

The delivery member cover 5 is configured to be moved linearly relative to the housing 3 from a first position shown in Fig. 1 to a second position in which the delivery member cover 5 is received further in the housing 3.

The delivery member cover 5 is biased in the proximal direction towards the first position.

The medicament delivery device 1 is triggered to discharge medicament by movement of the delivery member cover 5 further into the housing 3 towards the second position.

Fig. 2 shows an exploded view of the medicament delivery device 1.

The housing 3 is provided with a first radial opening 3c and a second radial opening 3d. The first radial opening 3c is arranged proximally relative to the second radial opening 3d. The first radial opening 3c and the second radial opening 3d are axially aligned.

The medicament delivery device 1 may comprise a housing sleeve 7 configured to cover at least a portion of the housing 3. The housing sleeve 7 may for example cover the first radial opening 3c and the second radial opening 3d.

The delivery member cover 5 comprises a radially flexible and radially outwards extending attachment member 5a, such as a tab, configured to engage with the first radial opening 3c when the delivery member cover 5 is in the first position.

The attachment member 5a is inclined and presents a decreasing ramp surface in the distal direction and can thereby flex radially inwards and disengage from the first radial opening 3c when the delivery member cover 5 is subjected to an axial force in the distal direction larger than a threshold value.

The attachment member 5a is configured to engage with the second radial opening 3c when the delivery member cover 5 is in the second position. The delivery member cover 5 is thereby interlocked with the housing 3 in the second position. The delivery member cover 5 is thus in this example not able to return to the first position from the second position.

The delivery member cover 5 has distal ends, each forming a cam surface 5b.

The medicament delivery device 1 comprises a proximal resilient member 9 configured to bear against an inner surface of a proximal end 5c of the delivery member cover 5. The proximal resilient member 9 may be a spring such as a coil spring.

The medicament delivery device 1 comprises a proximal sleeve 11. The proximal sleeve 11 is configured to be moved linearly relative to the housing 3. The proximal sleeve 11 is biased in the distal direction by the proximal resilient member 9. The distal end of the proximal resilient member 9 may for example bear directly against a proximal radial surface of the proximal sleeve 11.

According to the example shown in Fig. 2, the proximal sleeve 11 comprises guide arms 11a extending in the distal direction towards the distal end 3b of the housing 3.

The medicament delivery device 1 comprises a holding sleeve 13. The holding sleeve 13 is configured to engage with the housing 3. The holding sleeve 13 is axially and rotationally fixed relative to the housing 3. The holding sleeve 13 comprises radially outwards extending structures 13a configured to engage with a respective third radial opening 3e of the housing 3 for fixing the holding sleeve 13 to the housing 13.

The holding sleeve 13 comprises radial openings 13b. Each radial opening 13b has a distal end surface which is a radial surface.

The proximal sleeve 11 is configured to bear against the holding sleeve 13. The proximal sleeve 11 is configured to move axially in the distal direction relative to the holding sleeve 13.

The holding sleeve 13 comprises axial grooves 13c configured to receive a respective one of the guide arms 11a. The guide arms 11a are configured to move in the axial grooves 13c when the proximal sleeve 11 is moved in the distal direction.

The medicament delivery device 1 can be loaded with a mechanically flexible medicament container structure 15. The medicament container structure 15 comprises a proximal liquid container 15a and a distal gas container 15b. The liquid container 15a is arranged proximally relative to the distal gas container 15b. The gas container 15b is in fluid communication with the liquid container 15a. The liquid container 15a may comprise a liquid medicament. The gas container 15b may for example comprise air or an inert gas.

The container structure 15 may also comprise a planar end portion 15c arranged distally relative to the distal gas container 15b.

The medicament delivery device 1 comprises a hinge structure 17. The hinge structure 17 comprises two oppositely arranged hinge arms 17a. The hinge arms 17a are hingedly connected via a joint 17b. The hinge arms 17a extend in the proximal direction towards the proximal end 3a of the housing 3.

The hinge structure 17 is configured to receive a portion of the medicament container structure 15 between the two hinge arms 17a. According to the present example, the gas container 15b is configured to be arranged between the two hinge arms 17a.

The hinge arms 17a are inclined in relation to a radial plane, and thus extend radially outwards and away from each other in the proximal direction, when the distal gas container 15b is provided between the hinge arms 17a before use.

The hinge structure 17 may for example be made of a single integral piece of a polymeric material.

The hinge structure 17 comprises a liquid container holder 17c. The liquid container holder 17c is arranged proximally relative to the two hinge arms 17a. The liquid container 15a is configured to be arranged in the liquid container holder 17c. In particular, the liquid container holder 17c has a chamber 17d configured to receive the liquid container 15a. The liquid container 15a is typically arranged axially fixed relative to the liquid container holder 17c.

The liquid container holder 17c has oppositely arranged radially flexible arms 17e forming part of a respective side surface of the chamber 17d. Each arm 17e has a radially outwards extending structure 17f. The radially outwards extending structures 17f are configured to engage with a respective one of the radial openings 13b of the holding sleeve 13. The hinge structure 17 is thus axially locked to the holding sleeve 13. The holding sleeve 13, in turn, is axially fixed relative to the housing 3. The hinge structure 17 is thus fixed axially relative to the housing.

Each radially outwards extending structure 17f may have an inclined surface configured to cooperate with a respective radial surface of the holding sleeve 13. The inclined surfaces may for example have a ramp shape with a decreasing inclination in the distal direction.

The medicament delivery device 1 comprises a hollow drive member or expulsion member 19. The drive member 19 may have a cylindrical body. The drive member 19 is configured to be moved axially inside the housing 3. During a medicament delivery operation, the drive member 19 is configured to be moved from a first axial position towards the proximal end 3a of the housing 3.

The drive member 19 has an inner diameter which is smaller than a radial dimension of the hinge arms 17a. The radial dimension of the hinge arms 17a is defined by the largest radial distance between the outer surfaces of two the oppositely arranged hinge arms 17a.

When the delivery member cover 5 is in the first position, the drive member 19 is arranged distally relative to the two hinge arms 17a, in the first axial position.

The drive member 19 is biased in the proximal direction. The drive member 19 is axially fixed relative to the housing 3, in the first axial position, when the delivery member cover 5 is in the first position.

The drive member 19 may have a locking member 19c configured to engage with the housing 3 when the delivery member cover 5 is in the first position.

The delivery member cover 5 is configured to cooperate with the drive member 19. When the delivery member cover 5 is moved from the first position towards the second position, linear movement of the delivery member cover 5 is transformed into rotational movement of the drive member 19. This causes the drive member 19 to be released from engagement with the housing 3, and to move in the proximal direction. The hinge arms 17a are thereby moved radially inwards by the drive member 19, mechanically deforming the gas container 15b, as will be explained in more detail in the following.

The drive member 19a may comprise radially outwards extending protrusions 19a configured to cooperate with a respective one of the cam surfaces 5b to achieve rotation of the drive member 19a from an initial rotational position to a final rotational position as the delivery member cover 5 is moved from the first position to the second position.

The drive member 19 may for example have a distal end portion, wherein the distal end portion is provided with the locking member 19c. The locking member 19c may for example have two oppositely extending radial structures which when the drive member 19 is in the first rotational position are held by the housing 3 and which disengage from the housing 3 when the drive member 19 is rotated.

The medicament delivery device 1 comprises a distal resilient member 20. The distal resilient member 20 may for example be a spring, such as a coil spring. The distal resilient member 20 is configured to bias the drive member 19 in the proximal direction.

The drive member 19 has radial openings 19b. The radial openings 19b are provided in the cylindrical body of the drive member 19.

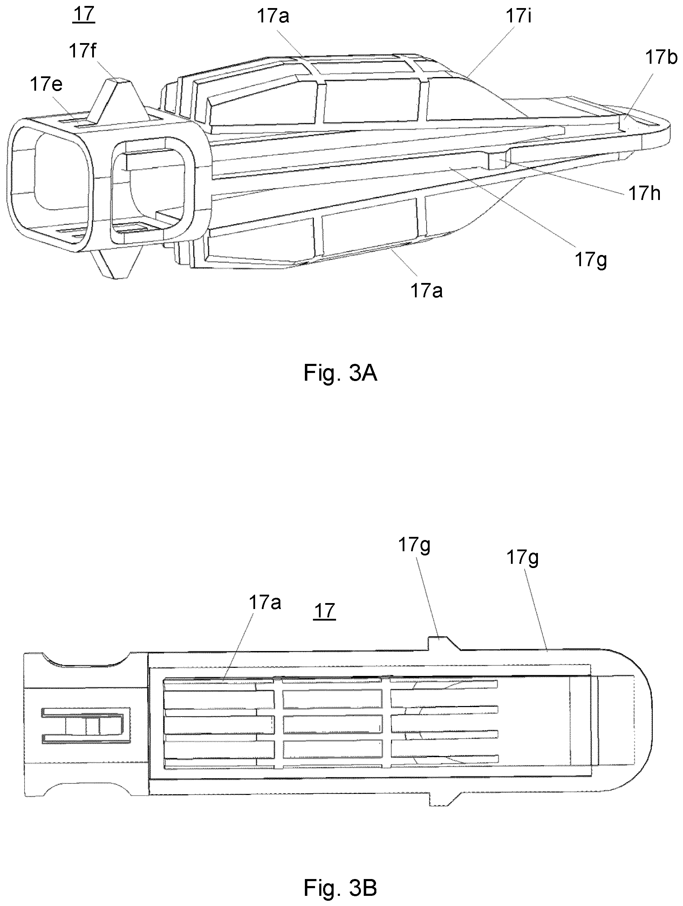

Fig. 3A shows a perspective view and Fig. 3B shows a top view of the hinge structure 17 in more detail. Each hinge arm 17a has an end portion 17i which tapers in the distal direction. The hinge arms 17a thus have a ramp shape, which increase in radial thickness in the proximal direction. When the drive member 19 is moved from the first axial position, its inner surface slides over the end portions 17i, causing the hinge arms 17a to be moved radially inwards towards each other as will be explained in more detail in the following.

The hinge structure 17 has a mechanically flexible frame 17g. The frame 17g is flexible radially. The frame 17g is provided with radially outwards extending protrusions 17h. The protrusions 17h are configured to bear against a proximal end of the drive member 19 for distancing the hinge arms 17a from contact with the drive member 19 when the drive member 19 is in the first axial position. The protrusions 17h have a ramp shape, increasing in radial thickness in the proximal direction.

When the drive member 19 is released from engagement with the housing 3, the frame 17g is flexed radially inwards and the hinge structure 17 is further received by the drive member 19. When the drive member 19 has moved in the proximal direction such that the radial openings 19b align axially with the protrusions 17h, the protrusions 17h are received by a respective one of the radial openings 19b. The hinge structure 17 is thereby interlocked with the drive member 19.

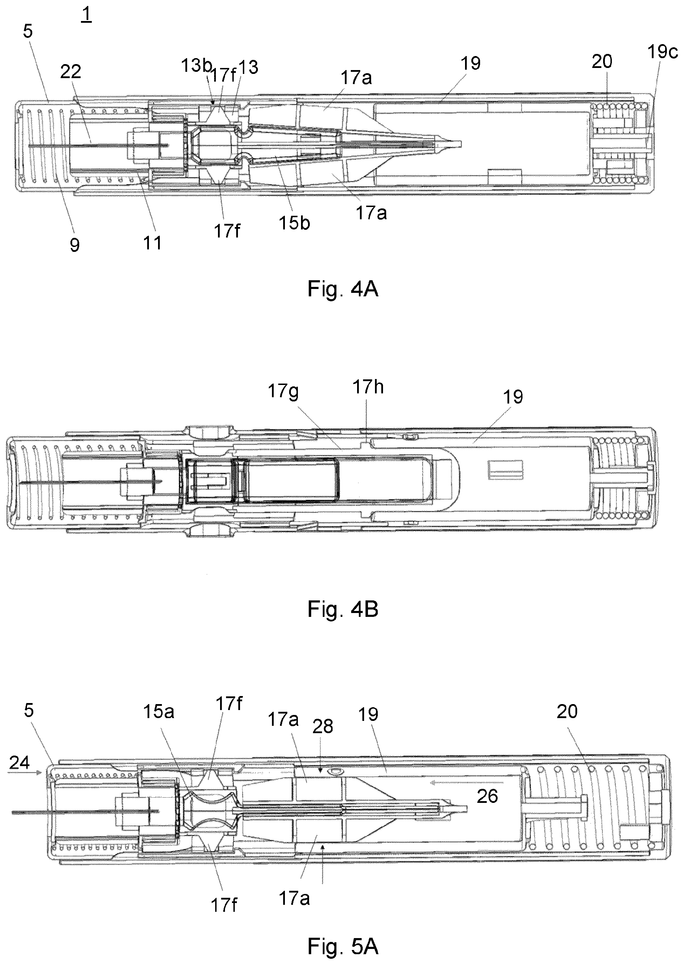

The operation of the medicament delivery device 1 will now be described in more detail with reference to Figs 4A-7.

Fig. 4A shows a sectional view of the medicament delivery device 1 in a default state before medicament delivery.

The delivery member cover 5 is in the first position, biased in the proximal direction by the proximal resilient member 9. The delivery member cover 5 covers the delivery member 22 of the medicament container structure 15.

The radially outwards extending structures 17f of the arms 17e are arranged in a respective radial opening 13b of the holding sleeve 13. The holding sleeve 13 engages with the housing 3 and is axially fixed relative to the housing 3. The proximal sleeve 11, which bears against the holding sleeve 13 and which is distally biased by the proximal resilient member 9 is thus arranged axially fixed and so is the hinge structure 17.

The gas container 15b is arranged between the hinge arms 17a. Moreover, the drive member 19 is arranged in the first axial position distally relative to the tapering end portions of the hinge arms 17a. The drive member 19 engages with the housing 3. In the example, the locking member 19c engages with a distal end of the housing 3. The distal resilient member 20 is arranged distally relative to the hollow cylindrical body of the drive member 19, biasing the drive member 19 in the proximal direction.

Fig. 4B shows a sectional view in a plane at 90 degrees angle relative to the sectional view shown in Fig. 4A. It can be seen that the protrusions 17h bear against a proximal end of the drive member 19. The hinge structure 17 and the drive member 19 are thus fixed relative to each other in the default state.

Fig. 5A shows the medicament delivery device 1 during a medicament delivery operation. The medicament delivery device 1 is pressed against the site of injection by the operator, causing the delivery member cover 5 to move from the first position to the second position, as illustrated by the arrow 24. The delivery member 22 is hence exposed, penetrating the tissue.

The distal movement of the delivery member cover 5 causes the cam surfaces 5b of the delivery member cover 5 to cooperate with a respective one of the radially outwards extending protrusions 19a of the drive member 19. This results in rotation of the drive member 19. The drive member 19 therefore disengages from the housing 3 and is moved in the proximal direction, as shown by arrow 26, due to the biasing by the distal resilient member 20. This proximal movement causes the drive member 19 to move over the hinge arms 17a, which are moved in the radial direction towards each other, discharging the content of the distal gas container 15b into the liquid container 15a, pushing the content of the liquid container 15a through the delivery member 22.

At the beginning of medicament expulsion, the liquid container 15a is pressurised enough by the liquid medicament to withstand the radially inwards directed force applied by the oppositely arranged radially outwards extending structures 17f which, being provided on the arms 17e, are urged radially inwards by the biasing force from the proximal resilient member 9 provided onto the proximal sleeve 11. Once the content of the liquid container 15a has been discharged, the liquid container 15a will not be sufficiently pressurised to withstand the radial pressure provided by the radially outwards extending structures 17f.

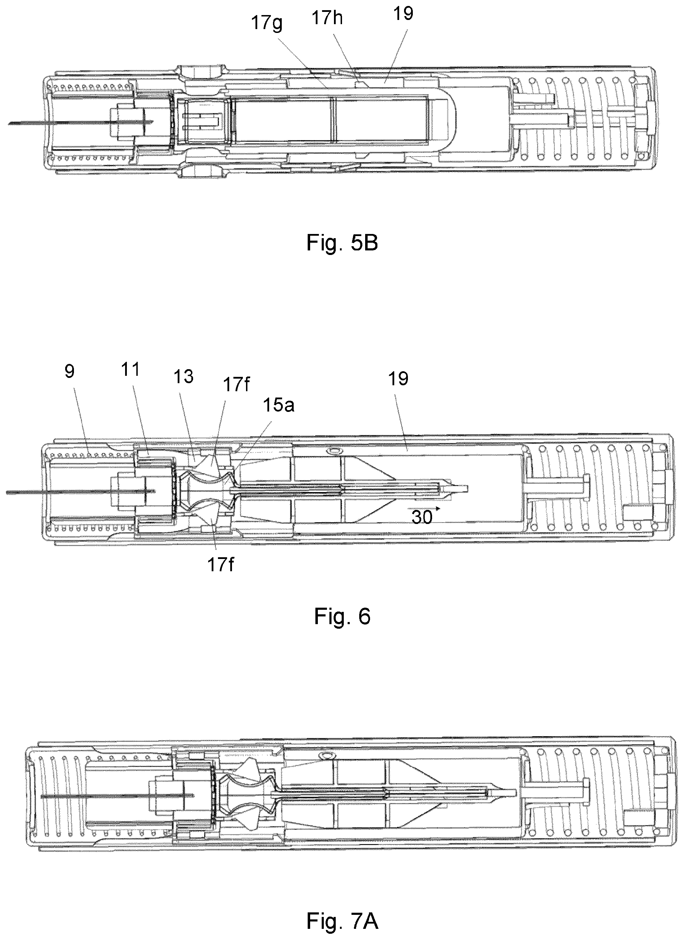

Fig. 5B shows a sectional view in a plane at 90 degrees angle relative to the sectional view shown in Fig. 5A. It can be seen that the protrusions 17h have been received by the drive member 19. The drawing is schematic, and in reality, the frame 17g would flex radially inwards and the protrusions 17h would bear against the inner surface of the drive member 19.

Fig. 6 shows the medicament delivery device 1 when the radially outwards extending structures 17f have collapsed over the liquid container 15a which has been emptied or essentially emptied of liquid. As a result, the hinge structure 17 is released from engagement with the holding sleeve 13. The proximal sleeve 11 will thus be moved in the distal direction, also moving the hinge structure 17 in the distal direction. The hinge structure 17 is thus moved further into the drive member 19, as shown by arrow 30, to obtain a final axial position. The hinge structure 17 also brings with it the medicament container structure 15, which is axially fixed relative to the hinge structure 17. The delivery member 22 is thus withdrawn into the housing 3, as shown in Fig. 7A, which shows a final state of the medicament delivery device 1, after use.

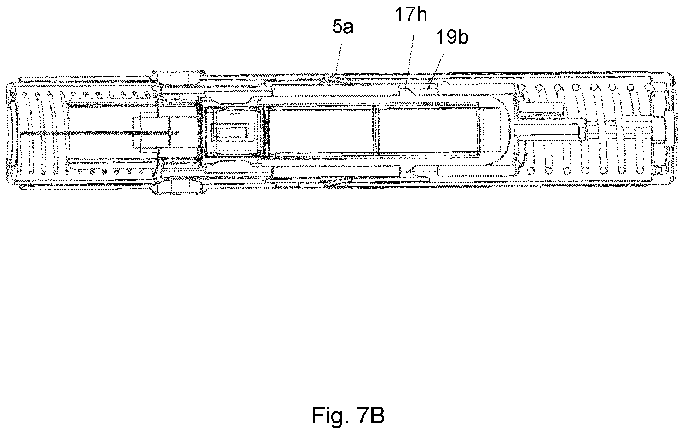

Fig. 7B shows a sectional view in a plane at 90 degrees angle relative to the sectional view shown in Fig. 7A. The protrusions 17h have engaged with the radial openings 19b of the drive member 19, axially interlocking the hinge structure 17 with the drive member 19. Moreover, the delivery member cover 5a is engaged with the second radial opening 3d of the housing 3. The delivery member cover 5a is thereby axially locked in the second position.

The medicament delivery device 1 may be disposed of in this final state, in which the delivery member 22 is protected inside the housing 3.

According to some variations, the drive member could for example be motor driven or manually driven instead of having an automatic spring-loaded structure. Moreover, it is envisaged that e.g. the hinge structure according to some examples could be provided without the radially outwards extending structures, and the medicament delivery device could thus be provided without the proximal sleeve and the holding sleeve.

The inventive concept has mainly been described above with reference to a few examples. However, as is readily appreciated by a person skilled in the art, other embodiments than the ones disclosed above are equally possible within the scope of the inventive concept, as defined by the appended claims.

## Claims

1. A medicament delivery device (1) for expelling medicament from a mechanically flexible medicament container structure (15), the medicament delivery device (1) comprising:
a housing (3) having a proximal end (3a) and a distal end (3b),
a hinge structure (17) having two hinge arms (17a), the hinge structure (17) being arranged in the housing (3) and configured to receive a portion of the medicament container structure (15) between the two hinge arms (17a),
**characterized in that** the medicament delivery device further comprises
a hollow drive member (19) biased towards the proximal end (3a) and arranged distally relative to the hinge arms (17a) in the housing (3), the drive member (19) having an inner diameter which is smaller than a radial dimension of the hinge arms (17a),
wherein the drive member (19) is configured to be released from a first axial position during a medicament delivery operation and move towards the proximal end (3a) of the housing (3), causing the drive member (19) to move radially over the hinge arms (17a) which thereby move radially inwards towards each other to mechanically deform the portion of the medicament container structure (15) for expulsion of medicament.

2. The medicament delivery device (1) as claimed in claim 1, wherein the hinge arms (17a) are directed towards the proximal end (3a) of the housing (3).

3. The medicament delivery device (1) as claimed in claim 2, wherein each hinge arm (17a) has an end portion (17i) that tapers in a direction towards the distal end (3b) of the housing (3).

4. The medicament delivery device (1) as claimed in any of the preceding claims, wherein the hinge structure (17) is biased towards the distal end (3b) of the housing (3), and wherein the hinge structure (17) has a frame (17g) provided with radially outwards extending protrusions (17h) configured to bear against a proximal end of the drive member (19) for distancing the hinge arms (17a) from contact with the drive member (19) when the drive member (19) is in the first axial position.

5. The medicament delivery device (1) as claimed in any of the preceding claims, comprising a distal resilient member (20) configured to bias the drive member (19) towards the proximal end (3a) of the housing (3).

6. The medicament delivery device (1) as claimed in any of the preceding claims, wherein the mechanically flexible medicament container structure (15) is a blow-fill-seal container.

7. The medicament delivery device (1) as claimed in any of the preceding claims, wherein
the mechanically flexible medicament container structure (15) has a proximal liquid container (15a) and a distal gas container (15b) in fluid communication with the liquid container (15a),
the hinge structure (17) has a liquid container holder (17c) biased towards the distal end (3b), the liquid container holder (17c) having a chamber (17d) configured to receive the liquid container (15a), and the liquid container holder (17c) having oppositely arranged radially flexible arms (17e) forming part of a respective side surface of the chamber (17d), each arm (17e) having a radially outwards extending structure (17f),
the hollow drive member (19) is configured to pressurise the gas container (15b) during the medicament delivery operation such that gas is expelled from the gas container (15b) into the liquid container (15a) to thereby expel liquid from the liquid container (15a),
the medicament delivery device (1) has a holding sleeve (13) arranged around the liquid container holder (17c) and being axially fixed relative to the housing (3),
the radially outwards extending structures (17f) are configured to be in contact with a respective radial surface of the holding sleeve (13) to prevent the liquid container holder (17c) from moving axially towards the distal end (3b), and
the arms (17e) are urged radially inwards against the liquid container (15a) by the biasing and the contact with the radial surfaces of the holding sleeve (13), wherein the arms (17e) are subjected to a radially outwards directed counterpressure by the liquid container (15a) when the liquid container (15a) is filled with liquid, and wherein the arms (17e) are configured to move radially inwards when the liquid container (15a) is emptied and its internal pressure is reduced, causing the radially outwards extending structures (17f) to be released from contact with the radial surfaces of the holding sleeve (13) and the liquid container holder (17c) to move towards the distal end (3b) inside the housing (3) and obtain a final axial position.

8. The medicament delivery device (1) as claimed in claim 7, wherein the radially outwards extending structures (17f) and/or the radial surfaces define inclined surfaces relative to a radial plane of the housing to facilitate radial inwards movement of the arms (17e).

9. The medicament delivery device (1) as claimed in claim 7 or 8, wherein the holding sleeve (13) comprises radial openings (19b), wherein each radial surface is formed by a distal end surface of a respective opening (19b).

10. The medicament delivery device (1) as claimed in any of claims 7-9, comprising a proximal sleeve (11), and a proximal resilient member (9) biasing the proximal sleeve (11) against the liquid container holder (17c) in a direction towards the distal end (3b) of the housing (3), thereby biasing the liquid container holder (17c) towards the distal end (3b) of the housing (3).

11. The medicament delivery device (1) as claimed in any of claims 7-10, wherein the hinge structure (17) is configured to receive the gas container (15b) between the two hinge arms (17a),
wherein the liquid container holder (17c) forms a proximal end of the hinge structure (17), and
wherein the radially inwards movement of the hinge arms (17a) towards each other mechanically deforms the gas container (15b).

12. The medicament delivery device (1) as claimed in claim 11 when dependent on claim 4, wherein the radially outwards extending protrusions (17h) are provided by a radially flexible frame (17g), and the drive member (19) has radial openings (19b) configured to interlock with a respective one of the protrusions (17h) when the liquid container holder (17c) has reached its final axial position.

## Patentansprüche

1. Arzneimittelabgabevorrichtung (1) zum Ausstoßen eines Arzneimittels aus einer mechanisch flexiblen Arzneimittelbehälterstruktur (15), die Arzneimittelabgabevorrichtung (1) umfassend:
ein Gehäuse (3), das ein proximales Ende (3a) und ein distales Ende (3b) aufweist,
eine Scharnierstruktur (17), die zwei Scharnierarme (17a) aufweist, wobei die Scharnierstruktur (17) in dem Gehäuse (3) angeordnet und konfiguriert ist, um einen Abschnitt der Arzneimittelbehälterstruktur (15) zwischen den zwei Scharnierarmen (17a) aufzunehmen,
**dadurch gekennzeichnet, dass** die Arzneimittelabgabevorrichtung ferner umfasst
ein hohles Antriebselement (19), das zu dem proximalen Ende (3a) hin vorgespannt und distal relativ zu den Gelenkarmen (17a) in dem Gehäuse (3) angeordnet ist, wobei das Antriebselement (19) einen Innendurchmesser aufweist, der kleiner als eine radiale Abmessung der Gelenkarme (17a) ist,
wobei das Antriebselement (19) konfiguriert ist, um während eines Arzneimittelabgabevorgangs aus einer ersten axialen Position freigegeben zu werden und sich zu dem proximalen Ende (3a) des Gehäuses (3) hin zu bewegen, was das Antriebselement (19) veranlasst, sich radial über die Gelenkarme (17a) zu bewegen, die sich dadurch radial nach innen aufeinander zu bewegen, um den Abschnitt der Arzneimittelbehälterstruktur (15) für einen Ausstoß des Arzneimittels mechanisch zu verformen.

2. Arzneimittelabgabevorrichtung (1) nach Anspruch 1, wobei die Scharnierarme (17a) zu dem proximalen Ende (3a) des Gehäuses (3) hin gerichtet sind.

3. Arzneimittelabgabevorrichtung (1) nach Anspruch 2, wobei jeder Scharnierarm (17a) einen Endabschnitt (17i) aufweist, der sich in einer Richtung zu dem distalen Ende (3b) des Gehäuses (3) hin verjüngt.

4. Arzneimittelabgabevorrichtung (1) nach einem der vorstehenden Ansprüche, wobei die Scharnierstruktur (17) zu dem distalen Ende (3b) des Gehäuses (3) hin vorgespannt ist, und wobei die Scharnierstruktur (17) einen Rahmen (17g) aufweist, der mit sich radial nach außen erstreckenden Vorsprüngen (17h) versehen ist, die konfiguriert sind, um an einem proximalen Ende des Antriebselements (19) zum Beabstanden der Scharnierarme (17a) von einem Kontakt mit dem Antriebselement (19) anzuliegen, wenn sich das Antriebselement (19) in der ersten axialen Position befindet.

5. Arzneimittelabgabevorrichtung (1) nach einem der vorstehenden Ansprüche, umfassend ein distales elastisches Element (20), das konfiguriert ist, um das Antriebselement (19) zu dem proximalen Ende (3a) des Gehäuses (3) hin vorzuspannen.

6. Arzneimittelabgabevorrichtung (1) nach einem der vorstehenden Ansprüche, wobei die mechanisch flexible Arzneimittelbehälterstruktur (15) ein Blasform-, Füll- und Verschließbehälter ist.

7. Arzneimittelabgabevorrichtung (1) nach einem der vorstehenden Ansprüche, wobei
die mechanisch flexible Arzneimittelbehälterstruktur (15) einen proximalen Flüssigkeitsbehälter (15a) und einen distalen Gasbehälter (15b) in Fluidverbindung mit dem Flüssigkeitsbehälter (15a) aufweist,
die Scharnierstruktur (17) einen Flüssigkeitsbehälterhalter (17c) aufweist, der zu dem distalen Ende (3b) hin vorgespannt ist, wobei der Flüssigkeitsbehälterhalter (17c) eine Kammer (17d) aufweist, die konfiguriert ist, um den Flüssigkeitsbehälter (15a) aufzunehmen, und der Flüssigkeitsbehälterhalter (17c) gegenüberliegend angeordnete, radial flexible Arme (17e) aufweist, die einen Teil einer jeweiligen Seitenoberfläche der Kammer (17d) ausbilden, wobei jeder Arm (17e) eine sich radial nach außen erstreckende Struktur (17f) aufweist,
das hohle Antriebselement (19) konfiguriert ist, um den Gasbehälter (15b) während des Arzneimittelabgabevorgangs derart mit Druck zu beaufschlagen, dass Gas aus dem Gasbehälter (15b) in den Flüssigkeitsbehälter (15a) ausgetrieben wird, um dadurch Flüssigkeit aus dem Flüssigkeitsbehälter (15a) auszutreiben,
die Arzneimittelabgabevorrichtung (1) eine Haltehülse (13) aufweist, die um den Flüssigkeitsbehälterhalter (17c) angeordnet und relativ zu dem Gehäuse (3) axial fixiert ist,
die sich radial nach außen erstreckenden Strukturen (17f) konfiguriert sind, um mit einer jeweiligen radialen Oberfläche der Haltehülse (13) in Kontakt zu stehen, um zu verhindern, dass sich der Flüssigkeitsbehälterhalter (17c) axial zu dem distalen Ende (3b) hin bewegt, und
die Arme (17e) durch das Vorspannen und den Kontakt mit den radialen Oberflächen der Haltehülse (13) radial nach innen gegen den Flüssigkeitsbehälter (15a) gedrängt werden, wobei die Arme (17e) einem radial nach außen gerichteten Gegendruck durch den Flüssigkeitsbehälter (15a) ausgesetzt sind, wenn der Flüssigkeitsbehälter (15a) mit Flüssigkeit gefüllt ist, und wobei die Arme (17e) konfiguriert sind, um sich radial nach innen zu bewegen, wenn der Flüssigkeitsbehälter (15a) entleert wird und sein Innendruck verringert wird, was die sich radial nach außen erstreckenden Strukturen (17f) veranlasst, aus dem Kontakt mit den radialen Oberflächen der Haltehülse (13) gelöst zu werden und sich der Flüssigkeitsbehälterhalter (17c) zu dem distalen Ende (3b) im Inneren des Gehäuses (3) hin zu bewegen und eine axiale Endposition einzunehmen.

8. Arzneimittelabgabevorrichtung (1) nach Anspruch 7, wobei die sich radial nach außen erstreckenden Strukturen (17f) und/oder die radialen Oberflächen geneigte Oberflächen relativ zu einer radialen Ebene des Gehäuses definieren, um eine radiale Bewegung der Arme (17e) nach innen zu erleichtern.

9. Arzneimittelabgabevorrichtung (1) nach Anspruch 7 oder 8, wobei die Haltehülse (13) radiale Öffnungen (19b) umfasst, wobei jede radiale Oberfläche durch eine distale Endoberfläche einer jeweiligen Öffnung (19b) ausgebildet wird.

10. Arzneimittelabgabevorrichtung (1) nach einem der Ansprüche 7 bis 9, umfassend eine proximale Hülse (11) und ein proximales elastisches Element (9), das die proximale Hülse (11) gegen den Flüssigkeitsbehälterhalter (17c) in einer Richtung zu dem distalen Ende (3b) des Gehäuses (3) hin vorspannt, wodurch der Flüssigkeitsbehälterhalter (17c) zu dem distalen Ende (3b) des Gehäuses (3) hin vorgespannt wird.

11. Arzneimittelabgabevorrichtung (1) nach einem der Ansprüche 7 bis 10, wobei die Scharnierstruktur (17) konfiguriert ist, um den Gasbehälter (15b) zwischen den zwei Scharnierarmen (17a) aufzunehmen,
wobei der Flüssigkeitsbehälterhalter (17c) ein proximales Ende der Scharnierstruktur (17) ausbildet, und
wobei die radial nach innen gerichtete Bewegung der Gelenkarme (17a) aufeinander zu den Gasbehälter (15b) mechanisch verformt.

12. Arzneimittelabgabevorrichtung (1) nach Anspruch 11, in Abhängigkeit von Anspruch 4, wobei die sich radial nach außen erstreckenden Vorsprünge (17h) durch einen radial flexiblen Rahmen (17g) bereitgestellt sind und das Antriebselement (19) radiale Öffnungen (19b) aufweist, die konfiguriert sind, um sich mit einem jeweiligen der Vorsprünge (17h) zu verriegeln, wenn der Flüssigkeitsbehälterhalter (17c) seine axiale Endposition erreicht hat.

## Revendications

1. Dispositif d'administration de médicament (1) permettant d'expulser un médicament d'une structure de récipient de médicament (15) mécaniquement flexible, le dispositif d'administration de médicament (1) comprenant :
un boîtier (3) ayant une extrémité proximale (3a) et une extrémité distale (3b),
une structure d'articulation (17) ayant deux bras d'articulation (17a), la structure d'articulation (17) étant agencée dans le boîtier (3) et conçue pour recevoir une partie de la structure de récipient de médicament (15) entre les deux bras d'articulation (17a),
**caractérisé en ce que** le dispositif d'administration de médicament comprend en outre
un élément d'entraînement creux (19) sollicité vers l'extrémité proximale (3a) et agencé distalement par rapport aux bras d'articulation (17a) dans le boîtier (3), l'élément d'entraînement (19) ayant un diamètre interne qui est inférieur à une dimension radiale des bras d'articulation (17a),
dans lequel l'élément d'entraînement (19) est conçu pour être libéré d'une première position axiale pendant une opération d'administration de médicament et se déplacer vers l'extrémité proximale (3a) du boîtier (3), amenant l'élément d'entraînement (19) à se déplacer radialement par-dessus les bras d'articulation (17a) qui se déplacent de ce fait radialement vers l'intérieur l'un vers l'autre pour déformer mécaniquement la partie de la structure de récipient de médicament (15) pour une expulsion de médicament.

2. Dispositif d'administration de médicament (1) selon la revendication 1, dans lequel les bras d'articulation (17a) sont dirigés vers l'extrémité proximale (3a) du boîtier (3).

3. Dispositif d'administration de médicament (1) selon la revendication 2, dans lequel chaque bras d'articulation (17a) a une partie d'extrémité (17i) qui s'effile dans une direction vers l'extrémité distale (3b) du boîtier (3).

4. Dispositif d'administration de médicament (1) selon l'une quelconque des revendications précédentes, dans lequel la structure d'articulation (17) est sollicitée vers l'extrémité distale (3b) du boîtier (3), et dans lequel la structure d'articulation (17) a un cadre (17g) pourvu de saillies (17h) s'étendant radialement vers l'extérieur conçues pour s'appuyer contre une extrémité proximale de l'élément d'entraînement (19) pour éloigner les bras d'articulation (17a) d'un contact avec l'élément d'entraînement (19) lorsque l'élément d'entraînement (19) est dans la première position axiale.

5. Dispositif d'administration de médicament (1) selon l'une quelconque des revendications précédentes, comprenant un élément élastique distal (20) conçu pour solliciter l'élément d'entraînement (19) vers l'extrémité proximale (3a) du boîtier (3).

6. Dispositif d'administration de médicament (1) selon l'une quelconque des revendications précédentes, dans lequel la structure de récipient de médicament (15) mécaniquement flexible est un récipient à soufflage-remplissage-scellage.

7. Dispositif d'administration de médicament (1) selon l'une quelconque des revendications précédentes, dans lequel
la structure de récipient de médicament (15) mécaniquement flexible a un récipient de liquide proximal (15a) et un récipient de gaz distal (15b) en communication fluidique avec le récipient de liquide (15a),
la structure d'articulation (17) a un support de récipient de liquide (17c) sollicité vers l'extrémité distale (3b), le support de récipient de liquide (17c) ayant une chambre (17d) conçue pour recevoir le récipient de liquide (15a), et le support de récipient de liquide (17c) ayant des bras radialement flexibles (17e) agencés de manière opposée faisant partie d'une surface latérale respective de la chambre (17d), chaque bras (17e) ayant une structure s'étendant radialement vers l'extérieur (17f),
l'élément d'entraînement creux (19) est conçu pour mettre sous pression le récipient de gaz (15b) pendant l'opération d'administration de médicament de telle sorte que du gaz est expulsé du récipient de gaz (15b) dans le récipient de liquide (15a) pour expulser de ce fait du liquide du récipient de liquide (15a),
le dispositif d'administration de médicament (1) a un manchon de support (13) agencé autour du support de récipient de liquide (17c) et étant fixé axialement par rapport au boîtier (3),
les structures s'étendant radialement vers l'extérieur (17f) sont conçues pour être en contact avec une surface radiale respective du manchon de support (13) pour empêcher le support de récipient de liquide (17c) de se déplacer axialement vers l'extrémité distale (3b), et
les bras (17e) sont poussés radialement vers l'intérieur contre le récipient de liquide (15a) par la sollicitation et le contact avec les surfaces radiales du manchon de support (13), dans lequel les bras (17e) sont soumis à une contre-pression dirigée radialement vers l'extérieur par le récipient de liquide (15a) lorsque le récipient de liquide (15a) est rempli de liquide, et dans lequel les bras (17e) sont conçus pour se déplacer radialement vers l'intérieur lorsque le récipient de liquide (15a) est vidé et que sa pression interne est réduite, amenant les structures s'étendant radialement vers l'extérieur (17f) à être libérées d'un contact avec les surfaces radiales du manchon de support (13) et le support de récipient de liquide (17c) à se déplacer vers l'extrémité distale (3b) à l'intérieur du boîtier (3) et à obtenir une position axiale finale.

8. Dispositif d'administration de médicament (1) selon la revendication 7, dans lequel les structures s'étendant radialement vers l'extérieur (17f) et/ou les surfaces radiales définissent des surfaces inclinées par rapport à un plan radial du boîtier pour faciliter un déplacement radial vers l'intérieur des bras (17e).

9. Dispositif d'administration de médicament (1) selon la revendication 7 ou 8, dans lequel le manchon de support (13) comprend des ouvertures radiales (19b), dans lequel chaque surface radiale est formée par une surface d'extrémité distale d'une ouverture respective (19b).

10. Dispositif d'administration de médicament (1) selon l'une quelconque des revendications 7 à 9, comprenant un manchon proximal (11), et un élément élastique proximal (9) sollicitant le manchon proximal (11) contre le support de récipient de liquide (17c) dans une direction vers l'extrémité distale (3b) du boîtier (3), sollicitant de ce fait le support de récipient de liquide (17c) vers l'extrémité distale (3b) du boîtier (3).

11. Dispositif d'administration de médicament (1) selon l'une quelconque des revendications 7 à 10, dans lequel la structure d'articulation (17) est conçue pour recevoir le récipient de gaz (15b) entre les deux bras d'articulation (17a),
dans lequel le support de récipient de liquide (17c) forme une extrémité proximale de la structure d'articulation (17), et
dans lequel le déplacement radial vers l'intérieur des bras d'articulation (17a) l'un vers l'autre déforme mécaniquement le récipient de gaz (15b).

12. Dispositif d'administration de médicament (1) selon la revendication 11 prise en dépendance de la revendication 4, dans lequel les saillies (17h) s'étendant radialement vers l'extérieur sont fournies par un cadre radialement flexible (17g), et l'élément d'entraînement (19) a des ouvertures radiales (19b) conçues pour s'emboîter dans une saillie respective parmi les saillies (17h) lorsque le support de récipient de liquide (17c) a atteint sa position axiale finale.
